# EUROPEAN PATENT APPLICATION

(11) **EP 4 844 056 A1**
(43) Date of publication of application: **25.11.2026**
(21) Application number: 24923183.8
(22) Date of filing: 17.06.2024
(51) Int. Cl.: C12N 5/0735, C12N 5/071, C12N 5/079, A01K 67/0271

(54) **COMPOSITION AND METHOD FOR CULTURING SIGNAL ORGANIZER AND MICRO-ORGAN USING SAME**

(30) Priority: 06.02.2024 CN 202410171326
(71) Applicant: Tongji University, Shanghai 200092 (CN)
(72) Inventor: ZHANG, Xiaoqing, Shanghai 200092 (CN); LIU, Ling, Shanghai 200092 (CN); ZHOU, Shanshan, Shanghai 200092 (CN)
(74) Representative: Hirons, Daniel Stuart
(86) International application number: PCT/CN2024/099607
(87) International publication number: WO 2025/166963

(57) **Abstract**

A composition and a method for culturing a signal organizer and a micro-organ using same. The composition comprises FGF2, a BMP inhibitor, a WNT signal activator and a Notch pathway inhibitor. The method comprises culturing a human pluripotent stem cell by using the composition. By means of induction by the composition, a human signal organizer is generated, and the human signal organizer is capable of spontaneously inducing the surrounding human pluripotent stem cells to generate various organ precursors, so that a combination of various micro-organs is generated.

## Description

The present application claims the priority of Chinese Patent Application No. 2024101713264 filed on February 6, 2024. The contents of the application are incorporated herein by reference in their entireties.

### TECHNICAL FIELD

The present disclosure relates to the field of biotechnology and, in particular, to a composition and a method for culturing a signal organizer and a micro-organ using the same.

### BACKGROUND

How different types of cells are formed, and why some stem cells in human embryos eventually become neurons instead of muscle cells, while other stem cells develop into cartilage tissue rather than heart tissue, have always been issues urgently to be addressed by the scientific community. In the early 1920s, German embryologist Hans Spemann and his student Hilde Mangold began to explore how embryonic stem cells differentiate into any type of cell tissue in the body, including bone, brain, lung, liver, and so on. In a salamander embryo experiment conducted in 1924, Spemann and Mangold discovered a unique group of cells. When this group was transplanted into another salamander embryo, it induced nearby cells to form the rudiments of the brain and spinal cord, and the new embryo grew into a pair of conjoined salamander twins. Subsequently, scientists found similar groups of cells in other amphibian and fish embryos. These groups of cells play an important shaping role in the early structural development of tissues and are thus called "organizers". They emit molecular signals that cause other cells to grow and develop in specific ways. When an organizer is transplanted from one embryo to another, the organizer stimulates its new host to gradually develop a central nervous system including the spinal cord and brain, while the organizer itself develops into tissues such as the bones of the spine.

However, to observe a human organizer cell, scientists have to extend the culture time of the embryo to 15-16 days. But constrained by ethical norms and laws, scientists cannot use human embryos that have developed for more than 14 days for experiments, because this is a critical point at which the embryo can no longer divide, and the embryo can then be considered an individual. It is these ethical and related issues that limit direct experiments using human embryos. Therefore, scientists have begun actively seeking various analogous substitutes.

To verify whether it is feasible, Brivanlou and his team conducted a series of experiments using synthetic human embryos. They cultured a small cluster of cell tissue approximately one millimeter in diameter from human embryonic stem cells in the laboratory. Although these synthetic mimic tissues are far from natural embryos, they still contain many cells and tissues present in real human embryos and can serve as experimental substitutes. The researchers transplanted the synthetic embryos into live chicken embryos to conduct studies more relevant to human systems. When the human embryonic cells were transplanted into the avian host, the human cells began to induce the foundational development of the spine and nervous system; obviously, this behavior marked the formation of a true human "signal organizer".

In chicken embryos, a second body axis can be induced by the human signal organizer, followed by the development of the nervous system. As to whether the human signal organizer can also induce the generation of human spinal nerves and various organs and tissues, due to ethical concerns, this process would be allowed to occur *in vitro*, using pluripotent stem cells as tool cells that possess the potential to develop into various organs. However, how to obtain the signal organizer and induce the generation of various human organs has become the most technically challenging point.

Organogenesis generally refers to the process in the individual development of vertebrates in which an organ primordium further evolves into an organ. Through the organogenesis stage, various organs undergo morphogenesis and tissue differentiation, gradually acquiring specific forms and performing certain physiological functions. In 2014, Lancaster et al. systematically proposed the concept of organoids, i.e., stem cells or organ progenitor cells, during *in vitro* culturing, can undergo cell differentiation and lineage commitment processes similar to those *in vivo*, and self-organize to form 3D structures that possess partial spatial structure and specific functions of human organs. Subsequently, organoids derived from an increasing number of tissues, such as intestine, retina, brain, and kidney, have been developed.

However, existing relatively mature human-derived organoid systems only simulate certain specific functions of organs, such as the respiratory function of the lung, the absorptive function of the intestine, and the filtration function of the kidney, and have not yet achieved the goal of fully replacing therapies or replacing transplantation, nor do they have the morphology of complete organogenesis. Moreover, the screening of anti-tumor drugs using single-type organoids carries the risk of strong auxiliary effects on other healthy organs, and requires a long period of experimentation before clinical application. In addition, the application of these single-type organoids is mainly focused on later stages of development, and their utility for drug screening during early pregnancy (i.e., the implantation period) is minimal.

Currently, according to technical research on early human embryos as shown in domestic and international journals, there is no relevant research on human signal organizers inducing the generation of multiple organs in humans. An article recently published abroad, titled "Self-organization of a human organizer by combined Wnt and Nodal signaling" (Martyn, I, et al., Nature, 2018. 558(7708): p. 132-135), only involved transplantation into chicken embryos without inducing various human organ tissues, and also involved planar monolayer cells without the morphology of a "signal organizer". There are also some other technologies related to early embryos, but none of them are related to a human signal organizer. How to obtain a signal organizer and induce the generation of various human organs has become the most technically challenging point.

### SUMMARY

To overcome the defect in the prior art that it is difficult to obtain a signal organizer and induce the formation of various organs, the present disclosure provides a composition and a method for culturing a signal organizer and a micro-organ using the same. The present disclosure provides a model for research on embryonic development before and after implantation during early pregnancy, solves the problem of difficulty in obtaining embryos, provides a model platform for drug screening during early pregnancy, and provides a source for organ transplantation.

A first technical solution of the present disclosure is to provide a composition, including FGF2, a BMP inhibitor, a WNT signal activator, and a Notch pathway inhibitor.

In some preferred embodiments, the BMP inhibitor is CC, Noggin, or LDN193189, the WNT signal activator is Wnt3a or CHIR99021, and the Notch pathway inhibitor is Compound E.

In some more preferred embodiments, the composition includes the FGF2, the CHIR99021, the Compound E, and the LDN193189.

In some specific embodiments, a molar ratio of the FGF2, the CHIR99021, the Compound E, and the LDN193189 is preferably 0.00058:30:2:1.

A second technical solution of the present disclosure is to provide a method for preparing a signal organizer, including: culturing human pluripotent stem cells using the composition according to the first technical solution, thereby harvesting the signal organizer.

In some preferred embodiments, the method includes the following steps:
(i) thoroughly mixing a Matrigel matrix with cell clusters containing the human pluripotent stem cells, where the human pluripotent stem cells are, for example, hESCs; and
(ii) when the Matrigel matrix solidifies, adding a differentiation medium containing the composition to perform differentiation.

In some more preferred embodiments, in (i), a volume-to-number ratio of the Matrigel matrix to the cell clusters containing the human pluripotent stem cells is 1:(1-2), for example, approximately 100-200 cell clusters containing the pluripotent stem cells are encapsulated by 100 µL of the Matrigel matrix; a concentration of the Matrigel matrix is 50%-100%; and

in (ii), a basal medium is mixed with the composition according to the first technical solution to prepare the differentiation medium; the basal medium is prepared with, for example, DMEM/F12 (1:1) and Neurobasal medium at a ratio of 1:1, supplemented with 1× GlutaMAX, 1× N2, and 1× B27; and the differentiation is performed in an incubator at 37°C.

In some preferred embodiments, the cell clusters, before being mixed with the Matrigel matrix, undergo the following treatment steps:
1) adding Dispase to digest the cell clusters, for example, digestion at 37°C;
2) after the digestion is complete, discarding the Dispase, washing with the basal medium, and then adding the basal medium;
3) pipetting down the cell clusters, collecting them into a container, further pipetting the cell clusters to a diameter of 30-80 µm, for example 50 µm, taking 2 mL of a middle fraction of the cell clusters, centrifuging to remove a resulting supernatant, and mixing.

For example, after pipetting the cell clusters with a total of 10 mL of the medium and collecting them in a 50 mL centrifuge tube, allowing natural sedimentation for 30 s, then aspirating 2 mL of the cell clusters and transferring them to a 15 mL centrifuge tube, followed by centrifugation. The middle fraction of the cell clusters refers to, after natural sedimentation, due to gravity, the cell clusters at the bottom are relatively large, and the cell clusters at the top are relatively small, neither of which is of the optimal size, whereas the cell clusters in the middle fraction are of suitable size, with a diameter of approximately 50 µm.

In some more preferred embodiments, before 1), the method further includes washing the cell clusters containing the human pluripotent stem cells with the basal medium and discarding the basal medium.

In some further preferred embodiments, in 2), the digestion lasts for 1-5 min, for example 3 min; and
in some further preferred embodiments, in 3), the centrifugation is performed at 1,000-1,500 rpm for 1-5 min.

A third technical solution of the present disclosure provides a signal organizer obtained by culturing according to the method of the second technical solution.

A fourth technical solution of the present disclosure provides a method for preparing an organ precursor cell and/or micro-organ, including transplanting the signal organizer according to the third technical solution into a mammal, such as a mouse, to generate the organ precursor cell and/or micro-organ, where the transplantation is, for example, performed subcutaneously or under a renal capsule in the mouse.

In the present disclosure, the micro-organ is a structure that, utilizing the pluripotency of the human pluripotent stem cells, under the induction of the "signal organizer", is orderly assembled by characteristic cells *in vitro* or *in vivo* and possesses corresponding functions.

A fifth technical solution of the present disclosure provides use of the composition according to the first technical solution for preparing a formulation for generating a signal organizer, an organ precursor cell and/or micro-organ.

A sixth technical solution of the present disclosure provides an organ precursor cell and/or micro-organ, prepared by the method according to the fourth technical solution.

A seventh technical solution of the present disclosure provides the composition according to the first technical solution, used for preparing generation of a signal organizer, an organ precursor cell and/or micro-organ.

An eighth technical solution of the present disclosure provides the signal organizer according to the third technical solution, used for preparing generation of an organ precursor cell and/or micro-organ.

A ninth technical solution of the present disclosure provides a method for treating a disease, including transplanting the organ precursor cell and/or micro-organ according to the sixth technical solution into a subject in need thereof to treat the disease.

A tenth technical solution of the present disclosure provides use of the organ precursor cell and/or micro-organ according to the sixth technical solution in drug screening.

The above preferred conditions can be combined in any appropriate manner based on general knowledge in the field to obtain various preferred embodiments of the present disclosure.

The reagents and raw materials used in the present disclosure are all commercially available.

The positive progressive effects of the present disclosure are as follows:
the technology of the present disclosure covers the generation of a "signal organizer" (the primary technology of the present disclosure is to generate a "signal organizer" through the induction of a unique formulation of small chemical molecules, and the importance of the "signal organizer" lies in its ability to spontaneously signal and induce surrounding human pluripotent stem cells to generate various organ precursors, thereby generating a combination of micro-organs); under its induced tissue, more combinations of organ precursor cells are obtained; relatively more complete organ generation morphology is achieved; the developmental process of various organs is simulated; multiple organs can be generated, allowing more direct detection of the effects and impacts of drugs on various organs; and it covers a wide range of developmental periods, enabling direct drug screening during early pregnancy. At the same time, the present disclosure faithfully simulates the development of various human organs and obtains relatively complete organ morphology, such as nerves, kidneys, intestines, and bones, overcoming the difficulty in obtaining early human embryos and providing an experimental source for drug screening during early pregnancy.

### BRIEF DESCRIPTION OF THE DRAWINGS

FIG. 1 shows an immunofluorescence staining image of cell clusters on the first day of differentiation.
FIG. 2 shows an immunofluorescence staining image of the cell clusters on the second day of differentiation.
FIG. 3 shows an immunofluorescence staining image of the cell clusters on the fourth day of differentiation.
FIG. 4 shows an immunofluorescence staining image of the cell clusters on the seventh day of differentiation.
FIG. 5 shows a Hematoxylin and Eosin staining image of the cell clusters transplanted subcutaneously into a mouse for 90 days on the fourteenth day of differentiation.
FIG. 6A to FIG. 6F show comparative example images of a combination of four compounds.
FIG. 7 shows a statistical graph of the results of a protocol in which FGF2 was removed.
FIG. 8 shows an immunofluorescence staining image of cells with knockout of β-catenin, an important gene downstream of a WNT signal.

### DETAILED DESCRIPTION OF THE PREFERRED EMBODIMENTS

The following will further illustrate the present disclosure by means of examples, but does not thereby limit the present disclosure to the scope of the described examples. Experimental methods without indicating specific conditions in the following examples should be selected according to conventional methods and conditions, or according to product specifications.

### Example 1

Raw materials: Matrigel matrix, FGF2, CHIR99021, Compound E, LDN193189, human pluripotent stem cells, differentiation medium, DMEM/F12 (1:1) basal medium, Dispase, M-NSG mice, and surgical operation tools.
FGF2: Peprotech, 100-18B
CHIR99021: Selleck, CT99021
Compound E: MCE, HY-14176
LDN193189: STEMGENT, 040074
Dispase: Gibco, 17105041
Human pluripotent stem cells: WA09, WiCell Agreement No. 17-W0044

### Part I: Induction of Human Pluripotent Stem Cells to Generate a Signal Organizer

### Steps:

1) The basal medium for inducing differentiation and Dispase were placed in a water bath at 37°C for preheating 10 minutes in advance; a 200 µL yellow pipette tip was placed at - 20°C for pre-cooling 10 minutes in advance, and the Matrigel matrix was taken out of a freezer at -80°C and placed in an ice box at 4°C for thawing 12 hours in advance.
2) A six-well plate for culturing human pluripotent stem cells was taken out of an incubator at 37°C; the original basal medium of the human pluripotent stem cells was aspirated; the cells were washed once with 2 mL of DMEM/F12 (1:1) basal medium; 1 mL of Dispase was added; and the plate was placed in the incubator at 37°C for digestion for 3 min.
3) The digestion of the human pluripotent stem cells was observed under a microscope, and when the edges of the human pluripotent stem cell colonies began to curl, the digestion was considered complete.
4) After discarding the Dispase, the cells were washed once with 2 mL of DMEM/F12 (1:1) basal medium to remove residual Dispase, and then 1 mL of DMEM/F12 (1:1) basal medium was added to each well.
5) 10 mL of DMEM/F12 (1:1) basal medium was aspirated using a 10 mL pipette; for each well of human pluripotent stem cells, the pipette was used to scrape horizontally and vertically 5 times each in a crosswise manner; and the cell clusters from three wells were pipetted down and collected into a 50 mL centrifuge tube.
6) The pipette was adjusted to the mid-range, and the cells were expelled at an appropriate speed against the bottom of the 50 mL centrifuge tube at a certain angle to break the cell clusters into smaller pieces; after repeating a certain number of times, the cells were broken into clusters of about 50 µm in diameter; then, the 50 mL centrifuge tube was placed vertically for 30 s to allow large hESC clusters to settle, and 2 mL of the middle fraction of the cell clusters was aspirated and transferred to a 15 mL centrifuge tube.
7) The hESC clusters were pelleted by centrifugation at 1,100 rpm for 1 min, the supernatant was discarded, and about 20 µL of liquid was retained and mixed using a 200 µL pipette tip.
8) The thawed Matrigel matrix was taken out of the freezer, and 200 µL was aspirated using the pre-cooled pipette tip; the cells were thoroughly mixed into the Matrigel matrix in the 15 mL centrifuge tube, with care taken to avoid creating bubbles; and four drops were placed in a 6 cm low-adhesion Corning culture dish, which was then carefully placed in an incubator at 37°C for 10 min to allow the matrix to solidify.
9) While waiting for the matrix to solidify, the differentiation medium was prepared. 50 mL of basal medium was aspirated into a 50 µL centrifuge tube, and 50 µL of bFGF, 15 µL of CHIR99021, 10 µL of Compound E, and 5 µL of LDN193189 were added and mixed (at a molar ratio of 0.00058:30:2:1) to obtain the differentiation medium. The culture dish was taken out of the incubator, 10 mL of the prepared differentiation medium was added, and then the dish was returned to the incubator to complete differentiation.
10) For the first three days, 10 mL of the prepared differentiation medium containing the four small chemical molecules was changed daily. Because the matrix was fragile, careful aspiration against the wall with a Pasteur pipette was required. From the third day to the seventh day, the basal medium was changed. A 3D cell cluster encapsulated by the whole Matrigel matrix was obtained.

The basal medium was prepared by mixing DMEM/F12 (1:1) and Neurobasal medium at a ratio of 1:1, and was supplemented with 1× GlutaMAX (5 mL of stock solution was added to 500 mL of basal medium) as well as 1× N2 (5 mL of stock solution was added to 500 mL of basal medium) and 1× B27 (10 mL of stock solution was added to 500 mL of basal medium). The differentiation medium was the basal medium supplemented with the four small molecules (i.e., the composition of the present disclosure).

### Part II: Transplantation of Organ Precursor Cells into a Mouse to Generate Micro-Organs

1) After anesthetizing an SPF-grade M-NSG mouse, an incision of about 1 cm in size was made on the back of the mouse near its upper limb. Before making the incision, the surgical area was shaved with a razor, and then iodine was sprayed for disinfection.
2) The whole 3D cell cluster encapsulated by the Matrigel matrix was transplanted subcutaneously using flat forceps.
3) The incision was sutured, and after suturing, iodine was reapplied for disinfection.

### Experimental Results

### 1) Detection method: immunofluorescence staining of cells

### Detection indicators: NANOG protein (indicating cell pluripotency), FOXA2 protein (indicating the signal organizer), and F-actin protein (indicating the cytoskeleton)

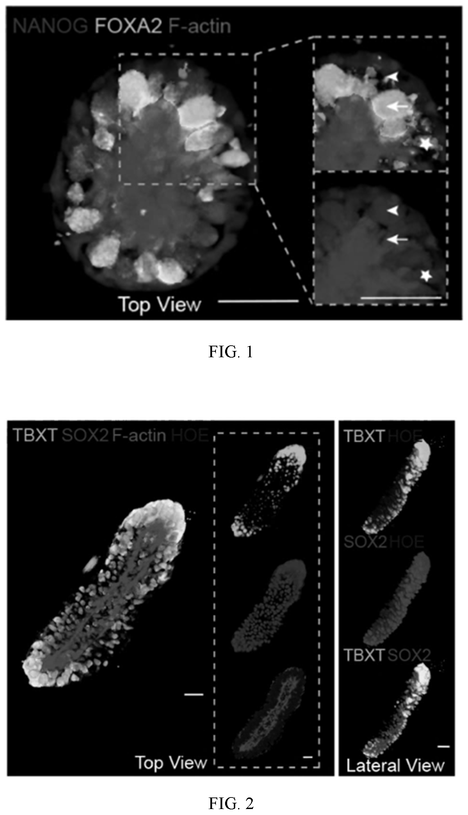

Result explanation: as shown in FIG. 1, the sample was cell clusters on the first day of differentiation, indicating that under the induction of the four small molecules, the pluripotency of human pluripotent stem cells were reduced, and they began to differentiate and transform toward a signal organizer. The arrowheads point to NANOG protein-positive and FOXA2 protein-negative cells, indicating stem cells; the arrows point to FOXA2 protein-positive and NANOG protein-positive cells, indicating "signal organizers"; and the stars point to NANOG protein-positive and FOXA2 protein-positive cells, indicating cells that are transitioning from stem cells toward "signal organizers".

### 2) Detection method: immunofluorescence staining of cells

### Detection indicators: TBX6 protein (indicating mesodermal precursor cells), and SOX2 protein (indicating neural precursor cells)

Result explanation: as shown in FIG. 2, the sample was cell clusters on the second day of differentiation, indicating that under the induction of the "signal organizer" generated on the first day, mesodermal and neuroectodermal precursor cells began to be generated.

### 3) Detection method: immunofluorescence staining of cells

### Detection indicators: TBX6 protein (indicating paraxial mesoderm cells), and SOX2 protein (indicating neural precursor cells)

The arrowheads point to TBX6 protein-positive cells, the arrows point to SOX2 protein-positive cells, and the stars indicate ZO-1 protein.

ZO-1 indicates tight junction protein, which is the line in the middle of the structure, indicating that these cells are polarized. HOE refers to Hoechst staining all cell nuclei.

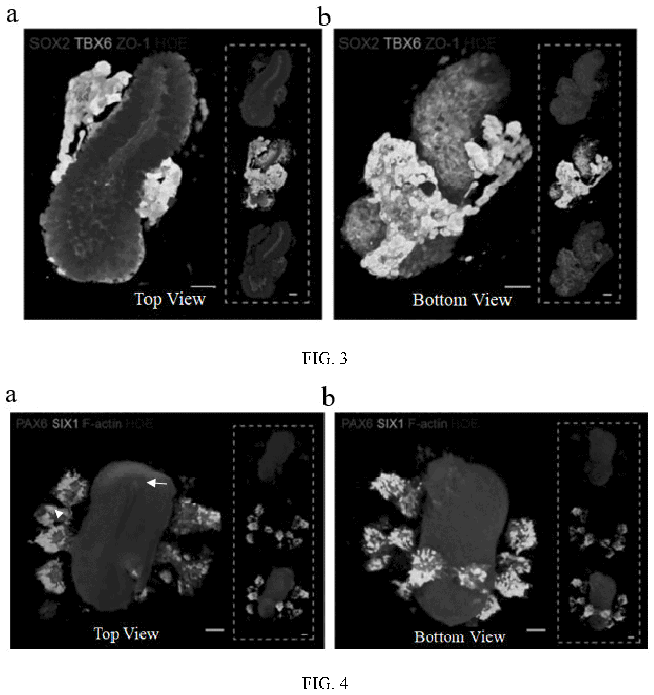

Result explanation: as shown in FIG. 3, the sample was cell clusters on the fourth day of differentiation, indicating that the mesodermal and ectodermal precursor cells generated on the second day further developed and differentiated, and the mesoderm migrated out of the structure to generate paraxial mesoderm surrounding the neural precursor cells. FIG. 3a is a top view, and FIG. 3b is a bottom view.

### 4) Detection method: immunofluorescence staining of cells

### Detection indicators: PAX6 protein (indicating spinal cord-derived neural precursor cells), and SIX1 protein (indicating somite-derived cells)

The arrowheads point to SIX1 protein-positive cells, and the arrows point to PAX6 protein-positive cells.

Result explanation: as shown in FIG. 4, the sample was cell clusters on the seventh day of differentiation, indicating that the paraxial mesoderm generated on the fourth day further developed and differentiated into somites, and the neural precursor cells in the middle further developed into spinal cord-derived precursor cells. FIG. 4a is a top view, and FIG. 4b is a bottom view.

### 5) Detection method: Hematoxylin and Eosin staining method

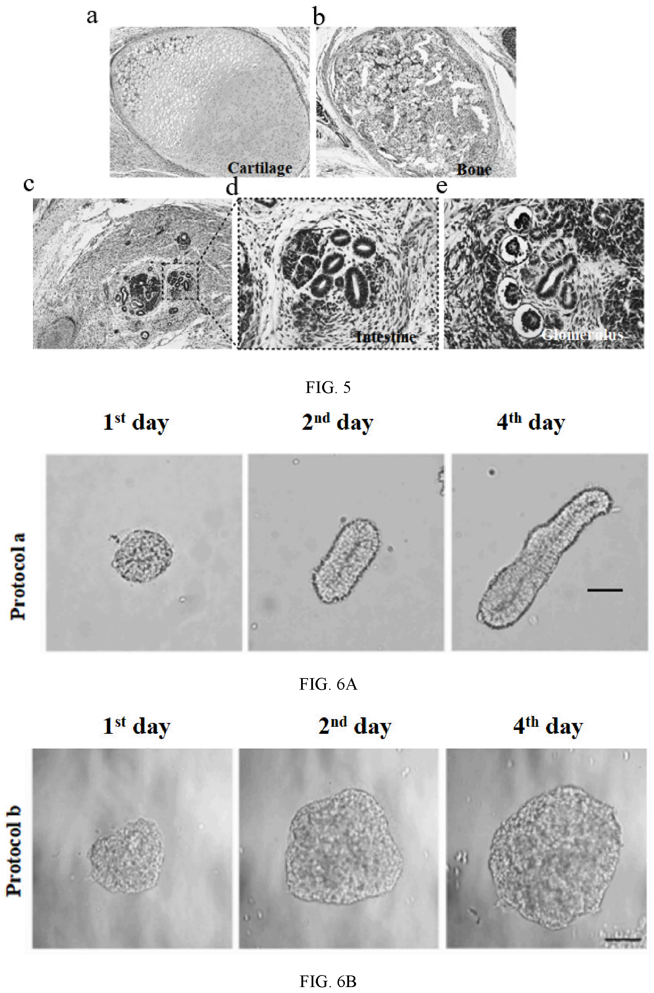

Result explanation: as shown in FIG. 5, the sample was cell clusters on the fourteenth day of differentiation (i.e., cell clusters of organ precursor cells), which were transplanted subcutaneously into a mouse (or under the renal capsule) for 90 days, indicating the generation of cartilage (part a), bone (part b), intestine (parts c and d, where d shows an enlarged view of c), and glomerulus (part e).

### 6) Detection method: observation under a microscope

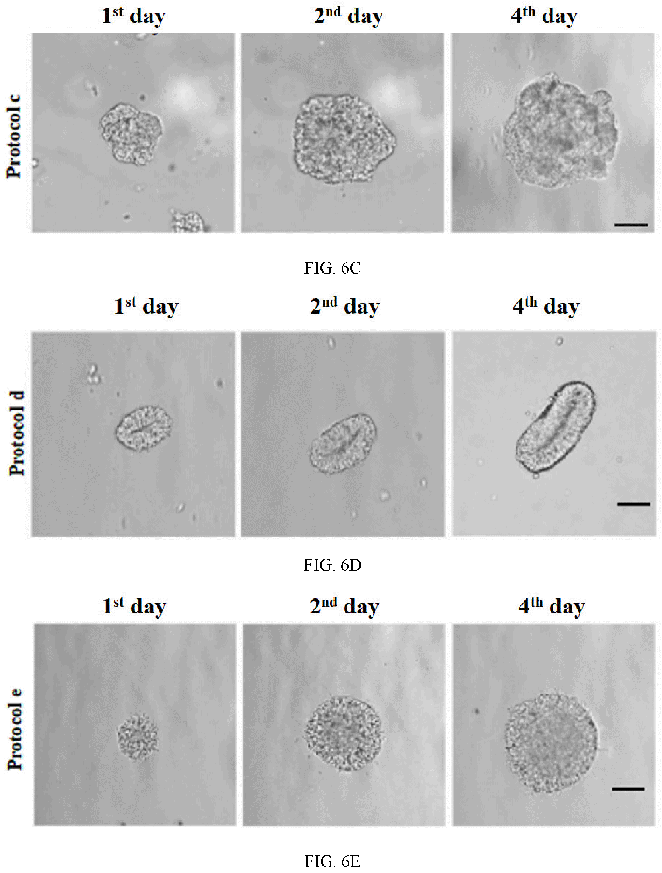

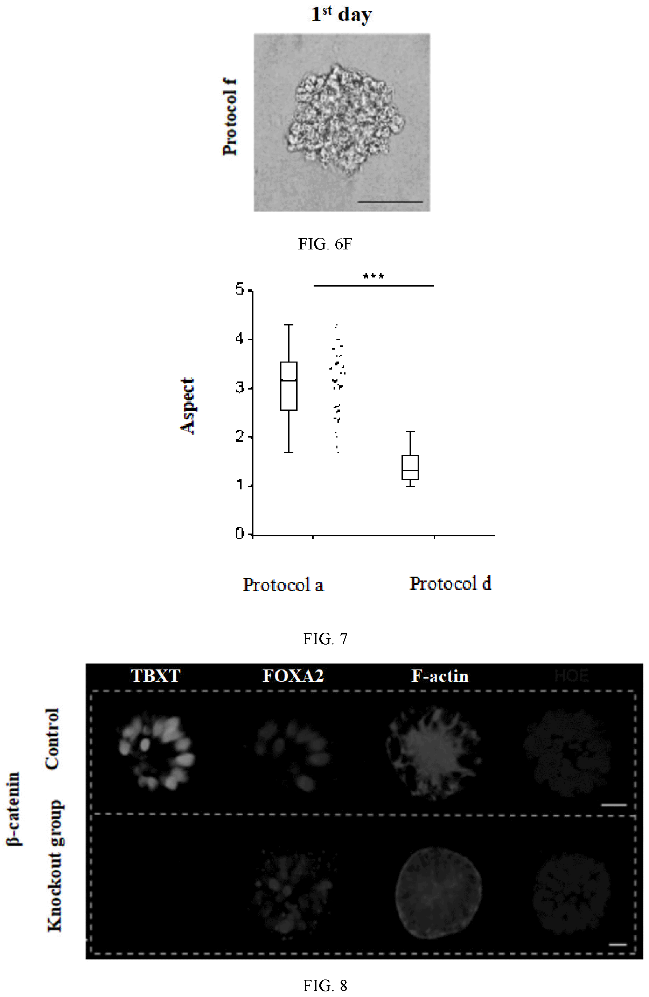

Result explanation: to fully demonstrate the necessity of the composition containing the four small chemical molecules, the following validation protocols for six combinations were designed, as shown in Table 1. Protocol a was a control group with the normal combination of four small molecules, and normal development was observed on the first, second, and fourth days, as shown in FIG. 6A. Protocols b, c, d, and e were designs with any one small molecule removed, and all resulted in abnormal development, as shown in FIGs. 6B, 6C, 6D, and 6E. Protocol f was designed by additionally adding a small molecule Activin A to activate the NODAL signaling pathway on the basis of the normal four small molecules, and the results showed abnormal development, as shown in FIG. 6F.

**Table 1 Validation Protocols for Small Molecule Combinations:**

| | FIG. 6A | FIG. 6B | FIG. 6C | FIG. 6D | FIG. 6E | FIG. 6F |
|---|---|---|---|---|---|---|
| Protocol | Protocol a | Protocol b | Protocol c | Protocol d | Protocol e | Protocol f |
| CHIR99021 | + | + | + | + | - | + |
| FGF2 | + | + | + | - | + | + |
| LDN193189 | + | + | - | + | + | + |
| Compound E | + | - | + | + | + | + |
| Activin A | - | - | - | - | - | + |

### 7) Detection method: biostatistics

Result explanation: the results in FIG. 6D showed abnormal development which was relatively similar to the control group; therefore, the aspect ratio of the structure was statistically analyzed. The results showed that the structure of Protocol d, from which FGF2 was removed, was relatively short, had a smaller aspect ratio, and exhibited a significant difference, as shown in FIG. 7.

### 8) Detection method: immunofluorescence staining

Result explanation: to further illustrate the importance of each signal, especially the WNT signal, in addition to removing the WNT activator CHIR99021 as shown in FIG. 6E, the important downstream β-catenin gene of the canonical WNT signaling pathway was further knocked out. After knockout, even under the condition of adding the four chemical compositions, the results still showed abnormal development, and the TBXT protein, one of the markers of the "signal organizer", was not expressed, further indicating the importance of the WNT signal, as shown in FIG. 8.

The above merely describes preferred embodiments for fully illustrating the present disclosure, and the scope of protection of the present disclosure is not limited thereto. Any equivalent substitutions or transformations made by those skilled in the art on the basis of the present disclosure fall within the scope of protection of the present disclosure.

Although the embodiments of the present disclosure are described above, those skilled in the art will understand that these embodiments are merely illustrative, and various changes or modifications can be made to these embodiments without departing from the principle and essence of embodiments. Therefore, the scope of protection of the present disclosure is defined by the appended claims.

## Claims

1. A composition, comprising FGF2, a BMP inhibitor, a WNT signal activator, and a Notch pathway inhibitor; preferably, the BMP inhibitor is preferably CC, Noggin, or LDN193189, the WNT signal activator is Wnt3a or CHIR99021, and the Notch pathway inhibitor is Compound E.

2. The composition according to claim 1, comprising the FGF2, the CHIR99021, the Compound E, and the LDN193189; preferably, a molar ratio of the FGF2, the CHIR99021, the Compound E, and the LDN193189 is preferably 0.00058:30:2:1.

3. A method for preparing a signal organizer, comprising: culturing a human pluripotent stem cell with the composition according to claim 1 or 2, thereby harvesting the signal organizer.

4. The method according to claim 3, comprising the following steps:
(i) thoroughly mixing a Matrigel matrix with a cell cluster containing the human pluripotent stem cell, wherein the human pluripotent stem cell is, for example, hESC; and
(ii) when the Matrigel matrix solidifies, adding a differentiation medium containing the composition to perform differentiation.

5. The method according to claim 4, wherein in (i), a volume-to-number ratio of the Matrigel matrix to the cell clusters is 1:(1-2); a concentration of the Matrigel matrix is 50%-100%; and in (ii), a basal medium is mixed with the composition according to claim 1 or 2 to prepare the differentiation medium; the basal medium is prepared with, for example, DMEM/F12 (1:1) and Neurobasal medium at a ratio of 1:1, supplemented with 1× GlutaMAX, 1× N2, and 1× B27; and the differentiation is performed in an incubator at 37°C.

6. The method according to claim 4 or 5, wherein the cell cluster, before being mixed with the Matrigel matrix, is subject to the following treatment steps:
1) adding Dispase to digest the cell clusters, for example, digestion at 37°C;
2) after the digestion is complete, discarding the Dispase, washing with the basal medium, and then adding the basal medium;
3) pipetting down the cell clusters, collecting them into a container, further pipetting the cell clusters to a diameter of 30-80 µm, for example 50 µm, taking 2 mL of a middle fraction of the cell clusters, centrifuging to remove a resulting supernatant, and mixing.

7. The method according to claim 6, wherein before 1), the method further comprises washing the cell cluster containing the human pluripotent stem cell with the basal medium and discarding the basal medium;
in 2), the digestion lasts for 1-5 min, for example 3 min; and
in 3), the centrifugation is performed at 1,000-1,500 rpm for 1-5 min.

8. A signal organizer obtained by culturing according to the method of any one of claims 3 to 7.

9. A method for preparing an organ precursor cell and/or micro-organ, comprising transplanting the signal organizer according to claim 8 into a mammal, such as a mouse, to generate the organ precursor cell and/or micro-organ, wherein the transplantation is, for example, performed subcutaneously or under a renal capsule in the mouse.

10. Use of the composition according to claim 1 or 2 in the manufacture of a formulation for generating an organ precursor cell and/or micro-organ.

11. An organ precursor cell and/or micro-organ, prepared by the method according to claim 9.

12. The composition according to claim 1 or 2, for use in generation of an organ precursor cell and/or micro-organ.

13. The signal organizer according to claim 8, for use in preparing generation of an organ precursor cell and/or micro-organ.

14. A method for treating a disease, comprising transplanting the organ precursor cell and/or micro-organ according to claim 11 into a subject in need thereof to treat the disease.

15. Use of the organ precursor cell and/or micro-organ according to claim 11 in drug screening.
